# EUROPEAN PATENT APPLICATION

(11) **EP 4 509 655 A1**
(43) Date of publication of application: **19.02.2025**
(21) Application number: 23791490.8
(22) Date of filing: 06.02.2023
(51) Int. Cl.: D21C 9/08, D21F 7/00, D21H 21/02

(54) **ESTIMATION DEVICE, ESTIMATION SYSTEM, ESTIMATION PROGRAM, AND ESTIMATION METHOD**

(30) Priority: 19.04.2022 JP 2022069109
(71) Applicant: Kurita Water Industries Ltd., Tokyo 164-0001 (JP)
(72) Inventor: WADA, Satoshi, Tokyo 164-0001 (JP); MORITA, Ryohei, Tokyo 164-0001 (JP)
(74) Representative: TBK
(86) International application number: PCT/JP2023/003715
(87) International publication number: WO 2023/203834

(57) **Abstract**

[Problem] To provide an estimation device, an estimation system, an estimation program, and an estimation method that can estimate pitch production from water quality information which can easily be measured during work.

[Solution] One aspect of the present invention provides an estimation device for estimating the degree of pitch production in a water system, or the degree of hindrance due to pitch. This estimation device comprises a turbidity information acquisition unit, a relationship model information acquisition unit, and an estimation unit. The turbidity information acquisition unit acquires turbidity information including the turbidity of the water system. The relationship model information acquisition unit acquires relationship model information which was generated in advance, and which indicates the relationship between the degree of pitch production or the degree of hindrance due to pitch, and turbidity. The estimation unit estimates the degree of pitch production or the degree of hindrance due to pitch, on the basis of the turbidity information and the relationship model information.

## Description

### BACKGROUND

The present invention relates to an estimation device, an estimation system, an estimation program, and an estimation method.

### RELATED ART

In pulp slurry in a papermaking step, natural resins and gums liberated from wood, pulp, waste paper, broke and the like, as well as sticky foreign matter mainly composed of organic substances derived from additive chemicals used in a pulp and paper manufacturing step, adhere to the slurry and flocculate, thus coarsening and forming pitch. When the pitch is formed in this way, it may adhere to paper products, resulting in defects that reduce the quality of the paper products, or it may adhere to the manufacturing devices, causing stains that need to be cleaned, thereby lowering productivity.

Thus, in order to understand the troubles due to occurrence of pitch, the amount of sticky foreign matter in the pulp slurry is measured. Such methods include solvent extraction, a dyeing method, a filtrate turbidity method, a PCD measurement, etc. (see Patent Document 1 and Non-Patent Document 1).

### PRIOR ART DOCUMENTS

### Patent document

[Patent Document 1] JP 2015-187326 A

### [Non-patent literature]

[Non-Patent Document 1] Jiayi Chen, JAPAN TAPPI JOURNAL, Vol. 57, No. 9, p. 1283 (published in 2003).

### SUMMARY

### Problems to be Solved by Invention

Among the above-mentioned methods for measuring the amount of sticky foreign matter, the solvent extraction and the dyeing method take time to perform measurement, making it difficult to grasp the status of occurrence of pitch based on the measurement results in real time. In addition, the solvent extraction and the dyeing method involve operations that are difficult to automate for analysis, making it difficult to continuously measure the amount of sticky foreign matter. On the other hand, in the filtrate turbidity method and the PCD method, the measurement sample is obtained by filtering the pulp slurry, and thus the state of the sample differs from that of the actual pulp slurry. This may result in a decrease in the accuracy of the measurement. In addition, in the filtrate turbidity method and the PCD method, a step of preparing the filtrate involves operations that are difficult to automate, and thus continuous human resources are required.

In view of the above circumstances, the present disclosure provides an estimation device, an estimation system, an estimation program, and an estimation method, which can estimate occurrence of pitch from water quality information that can be easily measured during operation.

### Means for Solving Problems

According to one aspect of the present disclosure, there is provided an estimation device for estimating a degree of occurrence of pitch or a degree of trouble due to pitch in a water system. The estimation device comprises a turbidity information acquisition unit, a relationship model information acquisition unit, and an estimation unit. The turbidity information acquisition unit is configured to acquire turbidity information including turbidity of the water system.

The relationship model information acquisition unit is configured to acquire relationship model information created in advance that indicates a relationship between the degree of occurrence of pitch or the degree of trouble due to pitch and the turbidity. The estimation unit is configured to estimate the degree of occurrence of pitch or the degree of trouble due to pitch based on the turbidity information and the relationship model information.

The present disclosure may be provided in each of the following aspects.
(1) An estimation device for estimating a degree of occurrence of pitch or a degree of trouble due to pitch in a water system, comprising: a turbidity information acquisition unit configured to acquire turbidity information including turbidity of the water system; a relationship model information acquisition unit configured to acquire relationship model information created in advance that indicates a relationship between the degree of occurrence of pitch or the degree of trouble due to pitch and the turbidity; and an estimation unit configured to estimate the degree of occurrence of pitch or the degree of trouble due to pitch based on the turbidity information and the relationship model information.
(2) The estimation device according to (1), wherein: the relationship model is a model obtained by a regression analysis, a time-series analysis, a decision tree, a neural network, Bayes, clustering, or ensemble learning between the degree of occurrence of pitch or the degree of trouble due to pitch and the turbidity.
(3) The estimation device according to (1) or (2), wherein: the turbidity is arithmetic mean turbidity or weighted mean turbidity of turbidity of the water system during stirring and turbidity of the water system during standing.
(4) The estimation device according to any one of (1) to (3), wherein: the water system is a water system in a step of manufacturing a paper product.
(5) An estimation system for estimating a degree of occurrence of pitch or a degree of trouble due to pitch in a water system, comprising: a turbidity information acquisition unit configured to acquire turbidity information including turbidity of the water system; a relationship model information acquisition unit configured to acquire relationship model information created in advance that indicates a relationship between the degree of occurrence of pitch or the degree of trouble due to pitch and the turbidity; and an estimation unit configured to estimate the degree of occurrence of pitch or the degree of trouble due to pitch based on the turbidity information and the relationship model information.
(6) An estimation program for estimating a degree of occurrence of pitch or a degree of trouble due to pitch in a water system, the estimation program allowing a computer to function as: a turbidity information acquisition unit configured to acquire turbidity information including turbidity of the water system; a relationship model information acquisition unit configured to acquire relationship model information created in advance that indicates a relationship between the degree of occurrence of pitch or the degree of trouble due to pitch and the turbidity; and an estimation unit configured to estimate the degree of occurrence of pitch or the degree of trouble due to pitch based on the turbidity information and the relationship model information.
(7) An estimation method for estimating a degree of occurrence of pitch or a degree of trouble due to pitch in a water system, comprising: a turbidity information acquisition step of acquiring turbidity information including turbidity of the water system; a relationship model information acquisition step of acquiring relationship model information created in advance that indicates a relationship between the degree of occurrence of pitch or the degree of trouble due to pitch and the turbidity; and an estimation step of estimating the degree of occurrence of pitch or the degree of trouble due to pitch based on the turbidity information and the relationship model information.

Of course, the present disclosure is not limited to the above aspects.

According to the present disclosure, it is possible to estimate occurrence of pitch from water quality information that can be easily measured during operation.

### BRIEF DESCRIPTION OF DRAWINGS

FIG. 1 is a schematic diagram of an estimation system according to the present embodiment.
FIG. 2 is a schematic diagram showing a functional configuration of the estimation device according to the present embodiment.
FIG. 3 is a schematic diagram showing a hardware configuration of the estimation device according to the present embodiment.
FIG. 4 is a schematic diagram showing an example in which the estimation system according to the present embodiment is applied to a paper manufacturing apparatus.
FIG. 5 is a schematic diagram of a chemical feeding system incorporated into a papermaking step shown in FIG.
   4.
FIG. 6 is a flowchart of an estimation method according to the present embodiment.
FIG. 7 is a plot of the impurity area versus turbidity in a case where the pitch control agent was not added.
FIG. 8 is a plot of the impurity area versus turbidity in the case where the pitch control agent was added.

### DETAILED DESCRIPTION

Hereinafter, an embodiment of the present disclosure will be described with reference to drawings. Various components shown in the embodiment described below can be combined with each other.

A program for realizing software according to the present embodiment may be provided as a non-transitory computer-readable medium, provided so as to be downloadable from an external server, or provided so as to be run on an external computer such that functions thereof are realized on a client terminal (so-called cloud computing).

In addition, a "unit" as referred to in the present embodiment may include, for example, a combination of hardware resources implemented by a circuit in a broad sense and information processing by software which may be specifically realized by such hardware resources. Furthermore, while various kinds of information are handled in the present embodiment, for example, such information are to be represented by a physical value of a signal value representing a voltage or a current, a level of a signal value as an aggregate of binary bits constituted of 0 or 1, or a quantum superposition (a so-called quantum bit), and communication and calculations can be executed on the circuit in a broad sense.

The circuit in a broad sense is a circuit realized by properly combining at least a circuit, circuitry, a processor, a memory, and the like. In other words, a circuit includes an application specific integrated circuit (ASIC), a programmable logic device (e.g., simple programmable logic device (SPLD), a complex programmable logic device (CLPD), field programmable gate array (FPGA), and the like.

### <Estimation system>

An estimation system according to the present embodiment is an estimation device for estimating a degree of occurrence of pitch or a degree of trouble due to pitch in a water system. Specifically, the estimation system comprises a turbidity information acquisition unit, a relationship model information acquisition unit, and an estimation unit. Among these, the turbidity information acquisition unit acquires turbidity information including the turbidity of the water system. Furthermore, the relationship model information acquisition unit acquires relationship model information created in advance that indicates a relationship between the degree of occurrence of pitch or the degree of trouble due to pitch and the turbidity. In addition, the estimation unit estimates the degree of occurrence of pitch or the degree of trouble due to pitch based on the turbidity information and the relationship model information.

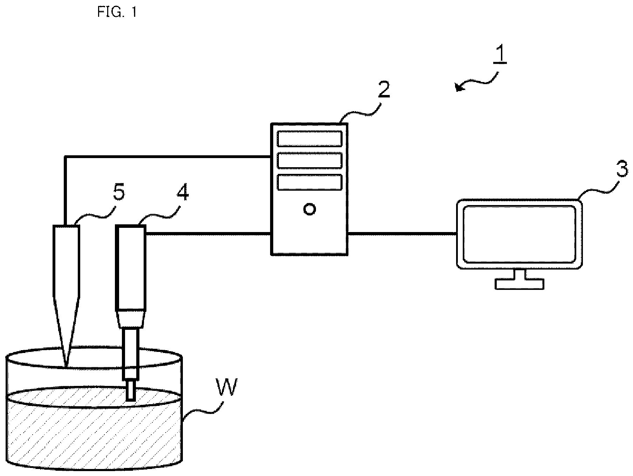

In addition, although not essential components, the estimation system according to the present embodiment may include one of or two or more of a relationship model creation unit, a water quality information measurement unit, a pitch control agent addition model creation unit, a pitch control inhibitor addition model information acquisition unit, an addition amount determination unit, an output unit, and a pitch control agent addition unit. Note that a prediction system including all of these units will be mainly explained with reference to FIG. 1 to be described below.

### [Functional configuration of estimation system]

FIG. 1 is a schematic diagram of an estimation system according to the present embodiment. The estimation system 1 comprises an estimation device 2, an output device 3, a turbidity information measurement device 4, and a pitch control agent addition device 5.

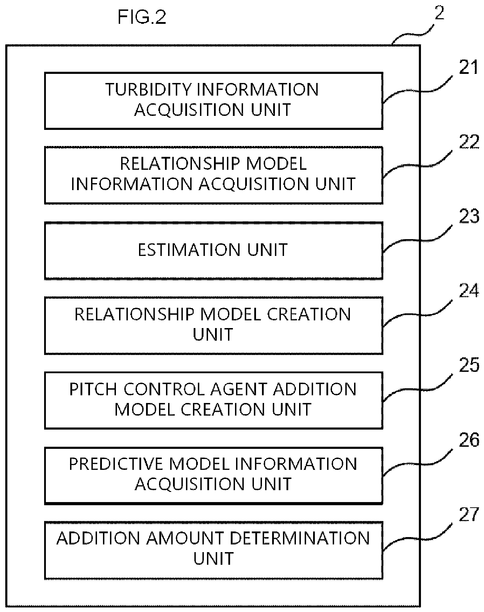

Among these, the estimation device 2 controls information processing for estimation in the estimation system 1. FIG. 2 is a schematic diagram showing a functional configuration of the estimation device according to the present embodiment. As shown in FIG. 2, the estimation device 2 according to the present embodiment is an estimation device for estimating a degree of occurrence of pitch or a degree of trouble due to pitch in a water system. The estimation device 2 comprises a turbidity information acquisition unit 21, a relationship model information acquisition unit 22, and an estimation unit 23. The estimation device 2 according to the present embodiment further comprises a relationship model creation unit 24, a pitch control agent addition model creation unit 25, a pitch control agent addition model information acquisition unit 26, and an addition amount determination unit 27. While the respective units are described herein as being included inside a single apparatus, alternatively, each unit may be included in a separate apparatus and may be included in multiple apparatuses. Furthermore, while the output device 3 is an example of the output unit, the turbidity information measurement device 4 is an example of a turbidity information measurement unit, and the pitch control agent addition device 5 is an example of the pitch control agent addition unit, a description will be hereinafter given without particularly distinguishing the devices from the units.

### [Functions of estimation system]

Hereinafter, a function of each unit of the estimation system 1 will be specifically described.

### [Turbidity information acquisition unit]

The turbidity information acquisition unit 21 acquires turbidity information including the turbidity of a water system W.

Note that the water system here is not limited to one existing in a single tank or a single flow path or one in which a continuous flow exists, and one with a plurality of tanks or flow paths, specifically, a water system in which a branch is present in a flow path, a water system with a confluence of a plurality of flow paths, a water system that is transferred in batch units from one tank to another, and a water system in which some kind of processing is performed in a mid-flow are also considered one water system.

As the turbidity of the water system, either the turbidity during stirring or the turbidity during standing may be used, but it is preferable to use both of them. In this way, in the case where both the turbidity during stirring and the turbidity during standing are used, it is preferable to use the arithmetic mean turbidity or the weighted mean turbidity of the two. In the case where the weighted mean turbidity is used, the ratio of the turbidity during stirring and the turbidity during standing is not particularly limited, for example, the ratio of the turbidity during stirring to the turbidity during standing is preferably 10:90 to 90:10, 20:80 to 80:20, 30:70 to 70:30, or 40:60 to 60:40.

The reasons why it is preferable to use both the turbidity during stirring and the turbidity during standing is explained below. The turbidity during stirring correlates to insoluble suspended solids. On the other hand, the turbidity during standing correlates to ash concentration. Since pitch components are present both in the insoluble suspended solids and the ash concentration, it is believed that measuring both turbidity will enable more accurate estimation.

### [Relationship model information acquisition unit]

The relationship model information acquisition unit 22 acquires relationship model information created in advance that indicates a relationship between the degree of occurrence of pitch or the degree of trouble due to pitch and the turbidity.

The relationship model is a model that is created in advance and that indicates the relationship between the degree of occurrence of pitch or the degree of trouble due to pitch and the turbidity. The term "in advance" refers to the time before the degree of occurrence of pitch or the degree of trouble due to pitch is estimated and may be the time before actual operation of the water system W, or during actual operation, as long as it is before the degree of occurrence of pitch or the degree of trouble due to pitch is estimated.

The relationship model is not particularly limited, but may include, for example, a function or a look-up table indicating a relationship between the degree of occurrence of pitch or the degree of trouble due to pitch and turbidity, or a trained model representing a relationship between the degree of occurrence of pitch or the degree of trouble due to pitch and turbidity.

### [Estimation unit]

The estimation unit 23 estimates a degree of occurrence of pitch or a degree of trouble due to pitch based on turbidity information and relationship model information.

Specifically, in the estimation unit 23, the degree of occurrence of pitch or the degree of trouble due to pitch is estimated or calculated by inputting the water system W turbidity information into the relationship model created and acquired in advance and by performing substitution, collation, or the like to the relationship model.

Note that the "degree of occurrence of pitch" includes, for example, the amount of occurrence of pitch and the impurity area.

The "degree of trouble due to pitch" includes, for example, in the case where the water system is a paper manufacturing water system, the number of pitch defects in paper manufactured by the water system, the area of the pitch defects, the number of times paper is broken due to pitch, the amount of broke due to pitch, the amount of pitch adhering to each part of a paper manufacturing apparatus, and the like.

### [Relationship model creation unit]

The relationship model creation unit 24 creates a relationship model. The relationship model is acquired by the relationship model information acquisition unit 22 and is used by the estimation unit 23 to estimate the degree of occurrence of pitch or the degree of trouble due to pitch.

The relationship model is created, for example, as follows Before estimating the degree of occurrence of pitch or the degree of trouble due to pitch, the degree of occurrence of pitch or the degree of trouble due to pitch is measured. In addition, in the same water system, the presence or absence of occurrence of pitch is confirmed or the degree of occurrence is measured. A data set of these parameters are prepared in plurality so that variations arise in the turbidity of the water system and the preliminary result by, for example, changing a date or time at which measurement is performed. Next, assuming that the preliminary result is a function of the turbidity of the water system, the function is compared with the preliminary result to determine a form and coefficients of the function, and a relationship model is constructed. At this time, when determining coefficients of the function by a comparison between the function of the turbidity of the water system and the preliminary result, a regression analysis method (a linear model, a generalized linear model, a generalized linear mixed model, ridge regression, lasso regression, an elastic net, support vector regression, projection pursuit regression, and the like), a time-series analysis (a VAR model, a SVAR model, an ARIMAX model, a SARIMAX model, a state space model, and the like), a decision tree (a decision tree, a regression tree, a random forest, XGBoost, and the like), a neural network (a simple perceptron, a multilayer perceptron, a DNN, a CNN, an RNN, an LSTM, and the like), Bayes (naive Bayes and the like), clustering (k-means, k-means ++, and the like), and ensemble learning (Boosting, Adaboost, and the like) can be used.

In one embodiment, the relationship model is preferably a model obtained from a regression analysis between the preliminary result and the turbidity of the water system. Note that the number of sample sets when performing a regression analysis is not particularly limited.

The creation of the relationship model is preferably performed in a same water system as the water system to be estimated. In addition, for example, in a case where the water quality of a water system changes significantly even in a same apparatus (for example, a case where pulp that is a papermaking raw material is changed or the like in a papermaking system at a papermaking plant), a relationship model with respect to the water system after the water quality changes is preferably created and used.

From such a viewpoint, the degree of occurrence of pitch or the degree of trouble due to pitch and the turbidity of the water system may be measured regularly or irregularly during operation of the water system W, and a relationship model may be created each time, or data may be added to update a relationship model.

Note that since the relationship model creation unit 24 is not an essential component, the creation of a relationship model may be manually performed by, for example, an operator or the like, without providing the relationship model creation unit 24.

### [Pitch control agent addition model creation unit]

The pitch control agent addition model creation unit 25 creates a pitch control agent addition model that is acquired in a predictive model information acquisition unit 26 described below and that is used to add an appropriate amount of pitch control agent in accordance with to the degree of occurrence of pitch or the degree of trouble due to pitch in the water system in a pitch control agent addition unit 5.

Here, the pitch control agent addition model refers to a model that indicates a relationship between the degree of occurrence of pitch or the degree of trouble due to pitch in a water system and the addition amount (e.g., minimum addition amount) of the pitch control agent.

The pitch control agent addition model is not particularly limited, but may include, for example, a function or a look-up table indicating a relationship between the degree of occurrence of pitch or the degree of trouble due to pitch in a water system and the addition amount of the pitch control agent or a trained model representing a relationship between the degree of occurrence of pitch or the degree of trouble due to pitch in a water system and the addition amount of the pitch control agent.

The pitch control agent to be added is not particularly limited as long as it can prevent the formation of the target pitch, and examples thereof may include surfactant, polymer, inorganic material, organic compound, oil content, starch, protein, enzyme, cyclodextrin, water-soluble cellulose, emulsifier, oxidant, and chelator.

Examples of the surfactant may include cationic surfactant, anionic surfactant, and non-ionic surfactant.

Examples of the polymer may include water-soluble polymer, cationic polymer, anionic polymer, ampholytic polymer, and non-ionic polymer. Examples of the polymer may include quaternary ammonium polymer, ester polymer, ether polymer, alcohol polymer, glycol polymer, ether ester polymer, methacrylic polymer, amide polymer, amine polymer, endocyclic nitrogen compound, polyvinyl alcohol, polyamide, polyether-ester amide, polyethyleneimine, polyamine/epihalohydrin, hydroxyalkyl cellulose, modified silicone, phenol resin, polyvinyl acetal resin, melamine resin, urethane resin, and poloxamer. Examples of the amide may include acrylamide. Examples of the amine may include alkylamine, alkylenediamine, and diallylamine.

Examples of the inorganic material may include alkali metal salt, aluminum compound, iron compound, calcium compound, talc, bentonite, zeolite, diatom earth, mica, white carbon, and inorganic acid.

Examples of the organic compound may include organic acid and alcohol. Examples of the organic acid may include phosphonic acid, gluconic acid, malic acid, citric acid, acetic acid, sulfonic acid, maleic acid, tartaric acid, lactic acid, and glycolic acid. Examples of the alcohol may include terpene alcohol. Examples of the endocyclic nitrogen compound may include pyrrolidone.

Examples of the oil content may include wax, mineral oil, vegetable oil, and animal oil.

The pitch control agent is commercially available from KURITA WATER INDUSTRIES LTD. under the product name "Spanplus". These pitch control agents may be used alone, or two or more kinds of pitch control agents may be used in combination.

Note that since the pitch control agent addition model creation unit 25 is not an essential component, the creation of a pitch control agent addition model may be manually performed by an operator or the like, without providing the pitch control agent addition model creation unit 25.

### [Pitch control agent addition model information acquisition unit]

The pitch control agent addition model information acquisition unit 26 acquires a pitch control agent addition model. The pitch control agent addition model may be, for example, one created by the pitch control agent addition model creation unit 25.

### [Addition amount determination unit]

The addition amount determination unit 27 determines the addition amount of the pitch control agent based on the degree of occurrence of pitch or the degree of trouble due to pitch in the water system W and the pitch control agent addition model. The degree of occurrence of pitch or the degree of trouble due to pitch in the water system W may be calculated by the estimation unit 23. The pitch control agent addition model may be one created by the pitch control agent addition model creation unit 25.

A minimum addition amount of the pitch control agent in the case where the degree of occurrence of pitch or the degree of trouble due to pitch in the water system W is a given degree is obtained from the degree of occurrence of pitch or the degree of trouble due to pitch in the water system W and the pitch control agent addition model. In the addition amount determination unit 27, the minimum addition amount may be adopted as it is as the addition amount, or the addition amount may be created by adding a safety factor or the like to the minimum addition amount.

### [Output unit]

The output unit 3 is configured to output at least the degree of occurrence of pitch or the degree of trouble due to pitch in the water system W calculated by the estimation unit 23.

The output unit 3 may, for example, display the degree of occurrence of pitch or the degree of trouble due to pitch in the water system W in a chronological manner (such as a graph of the degree of occurrence of pitch or the degree of trouble due to pitch in the water system W against time).

In addition, the output unit 3 may output a warning, for example, when the degree of occurrence of pitch or the degree of trouble due to pitch in the water system exceeds a certain threshold.

### [Turbidity information measurement unit]

The turbidity information measurement unit 4 measures turbidity information including the turbidity.

While only one turbidity information measurement device is described as the turbidity information measurement device 4 in Fig. 1 for the sake of convenience, the number of turbidity information measurement devices is not limited to this example and two or more turbidity information measurement devices may be used.

Specifically, as the turbidity information measurement device, a turbidimeter can be used. In particular, it is preferable to use an optical turbidimeter (e.g., an absorbance sensor, a reflected light sensor, or a transmitted light sensor) as the turbidity information measurement device.

The turbidity measured in this way may be directly received from the turbidity information measurement device, or may be input by an operator of the device, etc.

The water system to be an object of the estimation system and the like according to the present embodiment is not particularly limited as long as it is a water system in which pitch occurs and may be, for example, a water system in a step of manufacturing paper products. Specifically, a step of manufacturing paper products includes a cooking step, a washing step, a black liquorconcentration step, a causticizing step, and a papermaking step. In addition, as water systems other than a water system of the step of manufacturing paper products, examples of water systems to be an object include various piping, heat exchangers, storage tanks, kilns, and cleaning equipment.

### [Hardware configuration of estimation system]

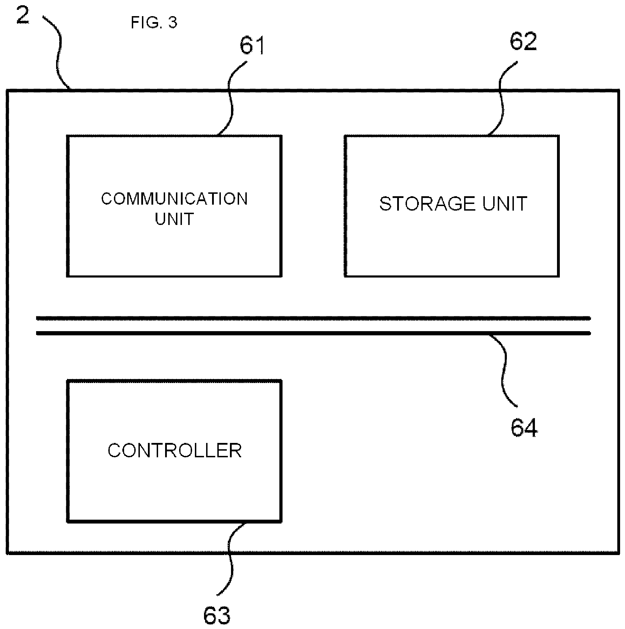

FIG. 3 is a schematic diagram showing a hardware configuration of the estimation device according to the present embodiment. As shown in FIG. 3, the estimation device 2 includes a communication unit 61, a storage unit 62, and a controller 63, and these components are electrically connected via a communication bus 64 inside the estimation device 2. Hereinafter, these components will be further described.

While the communication unit 61 is preferably wired communication means such as USB, IEEE 1394, Thunderbolt (registered trademark), and wired LAN network communication, the communication unit 61 can include wireless LAN network communication, mobile communication such as 3G/LTE/5G, Bluetooth (registered trademark) communication, and the like when necessary. In other words, more preferably, the communication unit 61 is implemented as an assembly of the plurality of communication means described above. Accordingly, exchange of information and commands is executed between the estimation device 2 and other devices capable of communicating with the estimation device 2.

The storage unit 62 stores various information defined by the description provided above. The storage unit 62 can be implemented as, for example, a storage device such as a solid state drive (SSD) or a memory such as a random access memory (RAM) which stores information (arguments, arrays, and the like) temporarily necessary in relation to calculations of a program. In addition, the storage unit 62 may be a combination thereof. Furthermore, the storage unit 62 stores various programs that can be read by the controller 63 described later.

The control unit 63 performs processing and control of overall operations related to the estimation device 2. This controller 63 is, for example, an unshown central processing unit (CPU). The controller 63 is configured to realize various functions related to the estimation device 2 by reading a predetermined program stored in the storage unit 62. In other words, information processing by software (stored in the storage unit 62) is specifically realized by hardware (the controller 63) and can be executed as each functional unit included in the controller 63 as shown in Fig. 3. Note that although the controller 63 is represented as a single unit in Fig. 3, actual configuration is not limited to this, and it may be configured to have a plurality of controllers 63 for each function. Moreover, a single controller may be combined with a plurality of controllers.

### Example of application of estimation system]

Hereinafter, a specific example in which the estimation system according to the present embodiment is applied to a paper manufacturing apparatus.

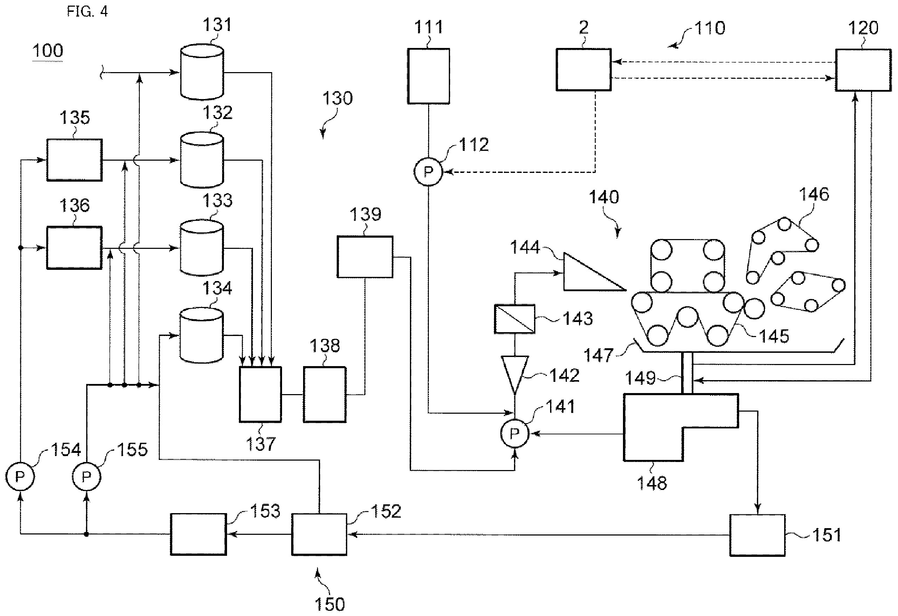

FIG. 4 is a schematic diagram showing an example in which the estimation system according to the present embodiment is applied to a paper manufacturing apparatus. The paper manufacturing apparatus 100 comprises a raw material system 130, a preparation/papermaking system 140, a recovery system 150, and a chemical feeding system 110.

The raw material system 130 comprises tanks 131, 132, 133, and 134 for storing papermaking raw materials, a mixing chest 137, a machine chest 138, and a seed box 139. On the other hand, the preparation/papermaking system 140 comprises a fan pump 141 that delivers pulp slurry supplied from the raw material system 130, a screen 142, a cleaner 143, an inlet 144, a wire part 145, and a press part 146. In addition, the preparation/papermaking system 140 is provided with a white water silo 148 for storing white water described below. The recovery system 150 comprises a seal pit 151, a recovery device 152, a recovery water tank 153, a disintegrating water pump 154, and a concentration-adjusting water pump 155.

The chemical feeding system 110 comprises a turbidity measurement unit 120 that measures the turbidity of white water, an estimation device 2 that controls the addition amount of internal chemicals to the pulp slurry, a tank 111, and a chemical feeding pump 112.

The raw material system 130 includes a chemical pulp tank 131, a recycled pulp tank 132, a broke tank 133 and a recovered raw material tank 134. The chemical pulp tank 131 contains chemical pulp such as softwood bleached kraft pulp (LBKP) and hardwood bleached kraft pulp (NBKP). The recycled pulp tank 132 contains deinked pulp (DIP) transferred from a deinking system and recycled pulp made by turning waste paper such as corrugated cardboard into a slurry using a waste paper pulper 135. The broke tank 133 contains pulp made by turning into a slurry by a broke pulper 136. The recovered raw material tank 134 contains pulp obtained by separating the white water into solid and liquid in a recovery device 152. Each pulp mentioned above is contained as a paper raw material, respectively.

Upstream of the chemical pulp tank 131, a device for manufacturing and supplying paper raw materials may be provided. That is, upstream of the chemical pulp tank 131, a digester for cooking wood chips, a device for bleaching pulp, a screen for removing foreign substances, etc. may be provided.

Note that the broke pulp produced after the press part 146 is supplied to the broke tank 133.

The waste paper pulper 135 and the broke pulper 136 are supplied with disintegrating water from the disintegrating water pump 154 for disintegrating the waste paper and broke. The recycled pulp, the broke pulp, the chemical pulp, and the recovered raw materials after being disintegrated by the pulpers 155 and 156 are combined with concentration-adjusting water that adjusts the concentration from the concentration-adjusting water pump 155 and are stored in each tank. In the present embodiment, recovered water from filtered white water is used as disintegrating water and concentration-adjusting water, but untreated filtered white water, fresh water, filtrate or squeezed water obtained by dehydrating the slurry of the raw material system 130, and surplus water from other steps may also be used.

The pulp stored in the chemical pulp tank 131, the recycled pulp tank 132, the broke tank 133, and the recovered raw material tank 134 is supplied to the mixing chest 137 in an appropriate ratio depending on the brand to be manufactured and is mixed in the mixing chest 137. The mixed pulp is transferred to the seed box 139 after papermaking chemicals are added in the machine chest 138.

The pulp stored in the seed box 139 is sequentially supplied with filtered white water from the white water silo 148 described later to the screen 142 and the cleaner 143 by the fan pump 141 of the preparation/papermaking system 140. After foreign substances are removed here, the pulp is supplied to the inlet 144. The inlet 144 supplies pulp to the wires of the wire part 145 at the proper concentration, speed, and angle. The supplied pulp is dehydrated in the wire part 145 and the press part 146, and then passed through a reel winder (not shown) to be manufactured into paper.

The water dehydrated from the pulp in the wire part 145 and the press part 146 is received as white water in a white water pit 147 located at the bottom of the wire part 145 and the press part 146. The white water received in the white water pit 147 is introduced into the white water silo 148 via a conduit 149 and is stored there. A part of the white water stored in the white water silo 148 is supplied to the fan pump 141, and the rest is supplied to the seal pit 151. The white water supplied to the fan pump 141 dilutes the pulp slurry in the preparation/papermaking system 140. The white water supplied to the seal pit 151 is transferred to the recovery device 152 where the white water is filtered and separated into solid and liquid. Then, the filtrate is collected in the recovery water tank 153.

Here, the pulp slurry supplied from the inlet 144 to the wire part 145 contains, as a suspending material, insoluble suspended solids (SS), i.e., pulp fibers of 20 µm or more in length, as well as fine ash (suspending material with a length of less than 20 µm) such as calcium carbonate and talc that are added as fillers. These insoluble suspended solids (SS) and ash are each captured by the wire part 145, and the rest is filtered down the wire and dispersed in the white water. Thus, the white water contains, as a suspending material, the insoluble suspended solids (SS), i.e., pulp fibers of 20 pm or more in length, as well as fine ash (suspended material with a length of less than 20 µm) such as calcium carbonate and talc that are added as fillers.

Hereinafter, the chemical feeding system 110 will be described.

Downstream of the fan pump 141, the tank 111 storing a pitch control agent is connected via the pump 112 (the tank 111 and the pump 112 correspond to the pitch control agent addition device 5). The pump 112 is electrically connected to the output side of the estimation device 2, and the turbidity measurement unit 120 is electrically connected to the input side of the estimation device 2.

A turbidity measurement unit 120 (corresponding to the turbidity information measurement device 4) collects white water from the conduit 149 connected to the white water pit 147, measures the turbidity of the white water, and transmits the measurement results to the estimation device 2. The estimation device 2 controls the injection amount of the pitch control agent in the tank 111 into the pulp slurry by operating the pump 112 in accordance with the transmitted measurement results.

Note that when the pitch control agent is added to the pulp slurry, it is shown here as being injected into the preparation/papermaking system 140 downstream of the fan pump 141. However, it is also possible to inject these internal chemicals into the raw material system 130 (e.g. the machine chest 138), or to inject these internal chemicals from the raw material system 130 into any location in the preparation/papermaking system 140, or to inject these internal chemicals into multiple locations thereof.

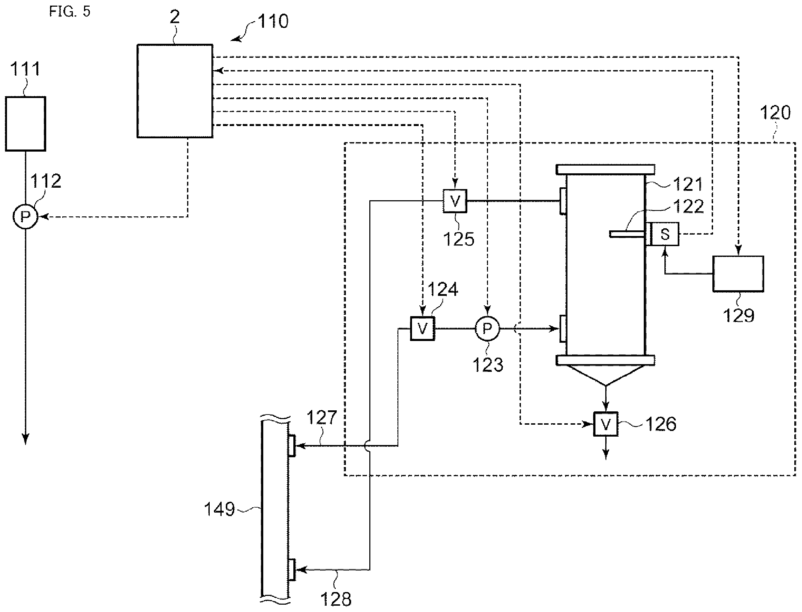

FIG. 5 is a schematic diagram of a chemical feeding system incorporated into the papermaking step shown in FIG. 4.

The turbidity measurement unit 120 is configured to collect white water from the conduit 149 in a predetermined procedure, store the collected white water in a measurement container 121, and measure the turbidity of the collected white water. Specifically, the turbidity measurement unit 120 comprises a supply system 127 provided with a sampling pump 123 for pumping up the white water flowing through the conduit 149 and supplying it to the measurement container 121, and a circulation system 128 that allows the white water exceeding the capacity of the measurement container 121 to flow out of the measurement container 121 and return to the conduit 149. These supply system 127 and circulation system 128 are provided with a supply valves 124 and a circulation valve 125, which are selectively opened and closed by the estimation device 2. The turbidity measurement unit 120 can collect the white water flowing through the conduit 149 in the measurement container 121 by controlling the opening and closing of these supply valve 124 and circulation valve 125 and controlling the operation of the sampling pump 123.

In the case where the sampling pump 123 is a positive-displacement type, the supply valve 124 is not necessary, and in the case where the measurement container 121 is an open system that allows the collected liquid to overflow, the circulation valve 125 is also not necessary. In addition, although it is assumed here that the white water is collected from the conduit 149, the white water may be collected directly from the white water pit 147.

Although it is preferable to use only one turbidity measurement unit 120 as shown in the present embodiment in terms of simplifying the system configuration, a plurality of turbidity measurement units may be used. In the case where a plurality of turbidity measurement units is employed, the turbidity during stirring and the turbidity during standing are measured in separate containers, so that both turbidity can be measured in parallel without waiting for the standing time.

The measurement container 121 has a depth of, for example, about 0.3 to 1.5 m and a capacity for storing 2 to 100 L, preferably about 3 to 5 L, of the white water, and includes a drain valve 126 forming a drainage system at its bottom. In addition, a turbidimeter 122 for measuring the turbidity of the upper part of the white water stored in the measurement container 121 is provided on the upper part of the inner wall surface of the measurement container 121, for example, at a position 50 to 200 mm below the water surface. Note that a water surface sensor (not shown) may be provided at the full water position on the inner wall of the measurement container 121 to detect that the water surface of the white water has reached the full water position.

A cleaning mechanism 129 is provided near the turbidimeter 122 and includes a water jet nozzle, a wiper (not shown) and the like for cleaning the periphery and the surface of the turbidimeter 122. This washing mechanism 129 measures the turbidity of the white water collected in the measurement container 121 by the turbidity sensor 122, and then opens the drain valve 126 to drain the white water. After that, the washing mechanism 129 selectively operates to clean the sensor surface of the turbidity sensor 122 and its surroundings, thereby playing a role in keeping the inside of the measurement container 121 clean.

Here, the supply speed of the supply system 127 (and the discharge speed of the circulation system 128) [L/sec] and the capacity [L] of the measurement container 121 are set so that the white water in the measurement container 121 is sufficiently stirred by the flow of white water flowing in from the supply system 127.

As described above, the estimation device 2 determines the injection amount of the pitch control agent to be injected into the pulp slurry based on the turbidity of the white water collected in the measurement container 121 and measured by the turbidimeter 122.

Such process for controlling the injection amount of the pitch control agent starts with an initial state in which the measurement container 121, the supply valve 124 and the circulation valve 125, and the drain valve 126 are set to "the measurement container 121: empty, the drain valve 126: open, and the supply valve 124 and the circulation valve 125: closed". First, the drain valve 126 is closed, the supply valve 124 and the circulation valve 125 are opened, and the sampling pump 123 is operated to pump up the white water from the conduit 149 via the supply system 127 and supply it to the measurement container 121. At the same time, with the start of supply of the white water to the measurement container 121, a timer (not shown) is activated to measure the supply time.

By supplying the white water, the amount of white water stored in the measurement container 121 increases, and when the white water stored in the measurement container 121 reaches full capacity, the white water exceeding the capacity of the measurement container 121 flows out through the circulation system 128 into the conduit 149. In this state, the amount of white water supplied to the measurement container 121 and the amount of white water flowing out from the measurement container 121 become equal, and the surface position of the white water in the measurement container 121 is maintained at the full water position. At this time, the white water stored in the measurement container 121 is in a stirred state as the white water is continuously supplied to the measurement container 121.

When the supply time reaches a preset predetermined time t1, that is, when the white water is supplied for a time t1 and the white water in the measurement container 121 becomes full, the turbidity of the white water is measured using the turbidimeter 122.

As described above, the white water stored in the measurement container 121 at this time is in a stirred state due to the continuous supply of white water, so the turbidity to be measured is the turbidity of the white water during stirring, and has a correlation with the insoluble suspended solids (SS) concentration of the white water as the turbidity equivalent to the insoluble suspended solids (SS).

Here, the predetermined time t1 is preset to the time required for the water surface position of the white water in the measurement container 121 to reach the predetermined full water height based on the capacity of the measurement container 121 and the supply speed of the white water. In addition, here, a timer is started when the supply of white water starts, and it is detected that the water surface position of the white water has reached the full water height after a predetermined time t1 has elapsed. However, instead of or in conjunction with the timer, it is also possible to detect that the water surface position of the white water has reached the full water height using a water level sensor (not shown) provided at the full water position of the measurement container 121.

When the measurement of turbidity of the white water during stirring is completed, the sampling pump 123 is then stopped, and the supply valve 124 and the circulation valve 125 are closed in a state that the drain valve 126 is closed. This stops the supply of white water to the measurement container 121 and the outflow of white water from the measurement container 121. Then, by stopping the supply and outflow of white water, the white water stored in the measurement container 121 is allowed to stand still.

At the same time as the white water stored in the measurement container 121 starts to stand still, continuous measurement of the turbidity of the white water is started using the turbidimeter 122, which is positioned to measure the turbidity at the top of the white water stored in the measurement container 121, as described above. This continuous measurement of the turbidity of the white water may be performed by sampling the measured values of the turbidimeter 122 at a predetermined sampling frequency (e.g., sampling frequency = 0.1 to 5 Hz).

Then, when a sufficient amount of time has elapsed after the start of the measurement and the continuous measured values of the turbidity of the white water have stabilized (for example, when the rate of change of the continuous measured values has become equal to or less than a predetermined value), the measurement is terminated, and the stable measured value is regarded as the turbidity of the white water during standing. The turbidity of the white water during standing has a correlation with the ash concentration of the white water as the turbidity equivalent to ash. Note that the lower the predetermined value of the rate of change that defines the standing state, the easier it is to obtain turbidity that is more highly correlated with the ash concentration. However, even if the value is excessively low, the increase in correlation is saturated, while the waiting time before measuring the turbidity during standing becomes longer. Thus, the specific predetermined value may be set appropriately based on the balance between the required control accuracy and efficiency, but may be set, for example, as the end point when the rate of decrease becomes 1 NTU/min or less.

Thereafter, the drain valve 126 is opened to drain all of the white water stored in the measurement container 121, and then the cleaning mechanism 239 is operated to clean the sensor surface of the turbidimeter 122 and its surroundings, thereby cleaning the inside of the measurement container 121, and returning it to the initial state described above.

Thereafter, the steps described above are continuously repeated in batches to continuously measure the turbidity of the white water during stirring and the turbidity of the white water during standing. That is, the chemical feeding system 110 can continuously and in real time measure the turbidity of the white water generated in the wire part 145 and the press part 146, during stirring and during standing.

Based on the turbidity during stirring and/or turbidity during standing obtained in this manner, the estimation device 2 calculates the addition amount of pitch control agent and adjusts the amount of internal chemical A to be injected into the pulp slurry by controlling the pump 112.

According to the estimation system 1 and the estimation device 2 described above, the degree of occurrence of pitch or the degree of trouble due to pitch in the water system W can be quantitatively measured in a simple manner.

### <Estimation program>

An estimation program according to the present embodiment is an estimation program for estimating a degree of occurrence of pitch or a degree of trouble due to pitch in a water system. Specifically, the estimation program allows the computer to function as a turbidity information acquisition unit, a relationship model information acquisition unit, and an estimation unit. Among these, the turbidity information acquisition unit is configured to acquire turbidity information including the turbidity of the water system. In addition, the relationship model information acquisition unit is configured to acquire relationship model information created in advance that indicates the relationship between the degree of occurrence of pitch or the degree of trouble due to pitch and the turbidity. Furthermore, the estimation unit is configured to estimate the degree of occurrence of pitch or the degree of trouble due to pitch based on the turbidity information and the relationship model information.

Note that, regarding the turbidity information acquisition unit, the relationship model information acquisition unit, and the estimation unit, since the same units as those of the estimation system described above can be used, a description thereof will be omitted here. In addition, the estimation system according to the present embodiment can further comprise a relationship model creation unit, a pitch control agent addition model creation unit, a pitch control agent addition model information acquisition unit, and an addition amount determination unit. For these units, the same units as those in the estimation system described above can be used, so a description thereof will be omitted here.

### <Estimation method>

An estimation method according to the present embodiment is an estimation method for estimating a degree of occurrence of pitch or a degree of trouble due to pitch in a water system. Specifically, the estimation method comprises a turbidity information acquisition step, a relationship model information acquisition step, and an estimation step. Among these, in the turbidity information acquisition step, the turbidity information including the turbidity of the water system is acquired. In addition, in the relationship model information acquisition step, relationship model information created in advance that indicates the relationship between the degree of occurrence of pitch or the degree of trouble due to pitch and the turbidity is acquired. Further in the estimation step, the degree of occurrence of pitch or the degree of trouble due to pitch is estimated based on the turbidity information and the relationship model information.

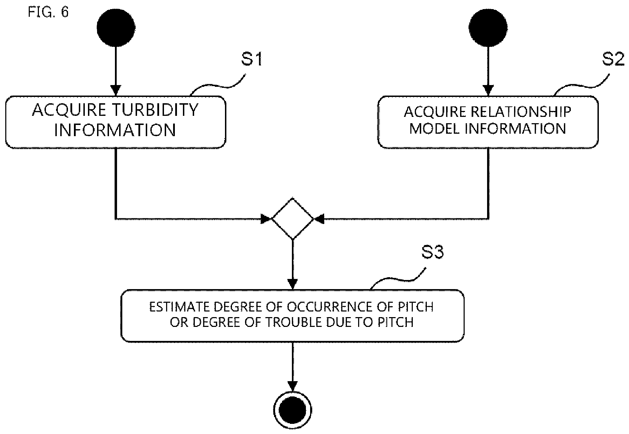

FIG. 6 is a flowchart of an estimation method according to the present embodiment. As shown in FIG. 6, in the estimation method according to the present embodiment, the turbidity information is acquired (turbidity information acquisition step S1) and the relationship model information is acquired (relationship model information acquisition step S2). Using the above information as input information, the degree of occurrence of pitch or the degree of trouble due to pitch is estimated (estimation step S3).

### [Example]

The present disclosure will be described below in more concrete terms by showing examples, but it is to be understood that the following examples are not intended to limit the present disclosure in any way whatsoever.

Cardboards collected in the city were disintegrated to a solid content concentration of approximately 2% using a disintegrator manufactured by FRANK-PTI GmbH, and approximately six liters were prepared as a sample. Six types of samples were created using different types of cardboard.

Next, each sample was divided into two portions, and a pitch control agent "SpanPlus 520" (manufactured by KURITA WATER INDUSTRIES LTD.) at 500 g/t solid content was added to one of the portions while stirring with a stirrer (EYELA) and stirring was continued for one minute. The other portion received no treatment.

For each of the six types of samples, samples with and without the addition of pitch control agent (12 types in total) were prepared by using a sheet machine papermaking device manufactured by KUMAGAI RIKI KOGYO Co., Ltd. to prepare wet paper so that the mass per unit area after drying would be 100 g/m². The wet paper was then dried using a rotary dryer manufactured by KUMAGAI RIKI KOGYO Co., Ltd., and the area of impurities on the paper surface was determined using an impurity measurement chart manufactured by Choyokai Co., Ltd. Ten sheets of wet paper were prepared, and the total area was determined.

The impurity area was measured in accordance with JIS P 8208 (1993), "Method for measuring foreign matter in pulp".

In addition, the same 12 types of samples were adjusted to a solid content concentration of 0.5%, and the stirring turbidity and the standing turbidity were measured using a turbidity measurement unit 120 shown in FIG. 5.

### <Results and Consideration>

The test results are shown in Table 1.

**[Table 1]**

| **SAMPLE** | **PITCH CONTROL AGENT** | **STIRRING TURBIDITY** | **STANDING TURBIDITY** | **AVERAGE OF STIRRING TURBIDITY AND STANDING TURBDITY** | **IMPURITY AREA** |
|---|---|---|---|---|---|
| 1 | NOT ADDED | 6880 | 2068 | 4474 | 192 |
| 2 | | 7570 | 2245 | 4908 | 313 |
| 3 | | 13872 | 5214 | 9543 | 482 |
| 4 | | 15529 | 1835 | 8682 | 557 |
| 5 | | 14396 | 4172 | 9284 | 885 |
| 6 | | 16816 | 5914 | 11365 | 1133 |
| 1 | ADDED | 5997 | 1455 | 3726 | 77 |
| 2 | | 7420 | 1607 | 4514 | 125 |
| 3 | | 13731 | 3642 | 8687 | 230 |
| 4 | | 15389 | 1511 | 8450 | 220 |
| 5 | | 14255 | 3010 | 8633 | 354 |
| 6 | | 16650 | 4433 | 10542 | 446 |

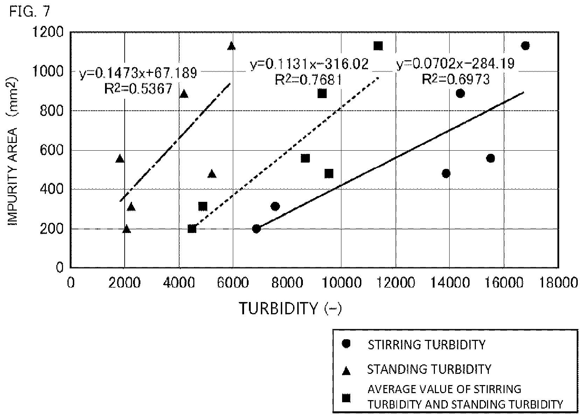

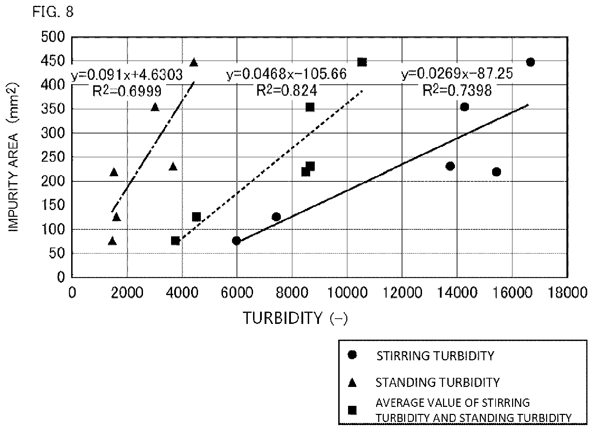

A plot of the impurity area versus turbidity was made for both the case where the pitch control agent was added and the case where the pitch control agent was not added, and a regression equation was obtained. FIG. 7 is a plot of the impurity area versus turbidity in the case where the pitch control agent was not added. FIG. 8 is a plot of the impurity area versus turbidity in the case where the pitch control agent was added.

As can be seen from FIG. 7, a high correlation coefficient was obtained in all cases: in a case where only the stirring turbidity was used, in a case where only the standing turbidity was used, and in a case where the average value of the stirring turbidity and the standing turbidity was used. In particular, a higher correlation coefficient was obtained in the case where the average values of the stirring turbidity and the standing turbidity was used compared to the case where only the stirring turbidity was used or the case where only the standing turbidity was used.

In addition, in the case where the pitch control agent was added as in FIG. 8, a high correlation coefficient was obtained similar to that in FIG. 7, and it was found that the correlation coefficient was not affected. Furthermore, it was also found that the impurity area could be reduced in the case where the pitch control agent was added.

### REFERENCE SIGNS LIST

- 1: Estimation system
- 2: Estimation device
- 21: Turbidity information acquisition unit
- 22: Relationship model information acquisition unit
- 23: Estimation unit
- 24: Relationship model creation unit
- 25: Pitch control agent addition model creation unit
- 26: Predictive model information acquisition unit
- 27: Addition amount determination unit
- 3: Output unit
- 4: Turbidity information measurement unit
- 5: Pitch control agent addition device
- 61: Communication unit
- 62: Storage unit
- 63: Controller
- 64: Communication bus
- 100: Paper manufacturing apparatus
- 110: Chemical feeding system
- 111: Tank
- 112: Pump
- 120: Turbidity measurement unit
- 121: Measurement container
- 122: Turbidimeter
- 123: Sampling pump
- 124: Supply valve
- 125: Circulation valve
- 126: Drain valve
- 127: Supply system
- 128: Circulation system
- 130: Raw material system
- 131: Chemical pulp tank
- 132: Recycled pulp tank
- 133: Broke tank
- 134: Recovered raw material tank
- 135: Waste paper pulper
- 136: Broke pulper
- 137: Mixing chest
- 138: Machine chest
- 139: Seed box
- 140: Preparation/papermaking system
- 141: Fan pump
- 142: Screen
- 143: Cleaner
- 144: Inlet
- 145: Wire part
- 146: Press part
- 147: White water pit
- 148: White water silo
- 149: Conduit
- 151: Seal pit
- 152: Recovery device
- 153: Recovery water tank
- 155, 156: Pulper

## Claims

1. An estimation device for estimating a degree of occurrence of pitch or a degree of trouble due to pitch in a water system, comprising:
a turbidity information acquisition unit configured to acquire turbidity information including turbidity of the water system;
a relationship model information acquisition unit configured to acquire relationship model information created in advance that indicates a relationship between the degree of occurrence of pitch or the degree of trouble due to pitch and the turbidity; and
an estimation unit configured to estimate the degree of occurrence of pitch or the degree of trouble due to pitch based on the turbidity information and the relationship model information.

2. The estimation device according to claim 1, wherein:
the relationship model is a model obtained by a regression analysis, a time-series analysis, a decision tree, a neural network, Bayes, clustering, or ensemble learning between the degree of occurrence of pitch or the degree of trouble due to pitch and the turbidity.

3. The estimation device according to claim 1, wherein:
the turbidity is arithmetic mean turbidity or weighted mean turbidity of turbidity of the water system during stirring and turbidity of the water system during standing.

4. The estimation device according to claim 1, wherein:
the water system is a water system in a step of manufacturing a paper product.

5. An estimation system for estimating a degree of occurrence of pitch or a degree of trouble due to pitch in a water system, comprising:
a turbidity information acquisition unit configured to acquire turbidity information including turbidity of the water system;
a relationship model information acquisition unit configured to acquire relationship model information created in advance that indicates a relationship between the degree of occurrence of pitch or the degree of trouble due to pitch and the turbidity; and
an estimation unit configured to estimate the degree of occurrence of pitch or the degree of trouble due to pitch based on the turbidity information and the relationship model information.

6. An estimation program for estimating a degree of occurrence of pitch or a degree of trouble due to pitch in a water system,
the estimation program allowing a computer to function as:
a turbidity information acquisition unit configured to acquire turbidity information including turbidity of the water system;
a relationship model information acquisition unit configured to acquire relationship model information created in advance that indicates a relationship between the degree of occurrence of pitch or the degree of trouble due to pitch and the turbidity; and
an estimation unit configured to estimate the degree of occurrence of pitch or the degree of trouble due to pitch based on the turbidity information and the relationship model information.

7. An estimation method for estimating a degree of occurrence of pitch or a degree of trouble due to pitch in a water system, comprising:
a turbidity information acquisition step of acquiring turbidity information including turbidity of the water system;
a relationship model information acquisition step of acquiring relationship model information created in advance that indicates a relationship between the degree of occurrence of pitch or the degree of trouble due to pitch and the turbidity; and
an estimation step of estimating the degree of occurrence of pitch or the degree of trouble due to pitch based on the turbidity information and the relationship model information.
